(19) Europäisches Patentamt
European Patent Office
Office européen des brevets

(11) Publication number: 0 107 464 B1

(12) # EUROPEAN PATENT SPECIFICATION

(45) Date of publication of patent specification: 08.01.86

(51) Int. Cl.[4]: C 07 F 9/38 // C07F9/65

(21) Application number: 83306278.9

(22) Date of filing: 17.10.83

(54) **Method for preparation of N-phosphonomethylglycine.**

(30) Priority: 18.10.82 US 435122

(43) Date of publication of application:
02.05.84 Bulletin 84/18

(45) Publication of the grant of the patent:
08.01.86 Bulletin 86/02

(84) Designated Contracting States:
AT BE CH DE FR GB IT LI NL SE

(56) References cited:
CH-A- 620 223
US-A-4 400 330

(73) Proprietor: STAUFFER CHEMICAL COMPANY
Westport Connecticut 06881 (US)

(72) Inventor: Bayer, Arthur Craig
3 Somerston Road
Yorktown Heights New York 105 (US)
Inventor: Bowler, Donald Joseph
2820 Minert Road
Concord California 94518 (US)
Inventor: Robbins, Jeffrey Dean
2 The Uplands
Berkeley California 94705 (US)

(74) Representative: Smith, Sydney et al
Elkington and Fife High Holborn House 52/54
High Holborn
London WC1V 6SH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 107 464 B1

**Description**

This invention relates to a novel method for the preparation of N-phosphonomethylglycine, a compound which is a known herbicide and plant growth regulator.

Herbicides are widely used by farmers, commercial agricultural companies, and other industries in order to increase crop yields for such staple crops as corn, soybeans and rice, and to eliminate weed growth along highways, railroad rights-of-way, and other areas. Herbicides are effective in killing or controlling unwanted weeds which compete for soil nutrients with the crop plants, and by reason of the fact they they kill weeds, are responsible for improving the aesthetic appearance of highway and railroad rights-of-way. There are a number of different types of herbicides presently sold commercially, and these fall into two general categories. The categories are pre-emergence and post-emergence herbicides. The pre-emergence herbicides are incorporated into the soil prior to the emergence of the weed plants from the soil, and the post-emergence herbicides are applied to plant surfaces after emergence of the weeds or other unwanted plants from the soil.

One of the earliest post-emergence herbicides used commercially was 2,4-D (2,4-dichlorophenoxyacetic acid). After a number of years of use of this and similar compounds such as 2,4,5-T (2,4,5-trichlorophenoxy acetic acid), it was found that certain decomposition products of these herbicides were long lasting and were not biodegradable. While there has been some dispute between governmental agències and commercial interests regarding the effects of residual products of 2,4-D, 2,4,5-T and similar compounds, the agencies nevertheless restricted the use of these herbicides in the United States some years ago. Since that time, efforts have been made to develop herbicides which are biodegradable into harmless residues within a relatively short time after their application.

One such compound, which has been found to be biodegradable, yet which is effective as a herbicide and plant growth regulator when employed at lower rates, is N-phosphonomethylglycine and various salts thereof. N-Phosphonomethylglycine and agriculturally effective salts have been approved for use by the U.S. Government, and, as a consequence, this herbicide has become extremely successful commercially.

N-Phosphonomethylglycine and certain salts are the only effective and approved post-emergence herbicides in the field. The present commercial compound is the isopropylame salt of N-phosphonomethlglycine and derivatives thereof.

In field use it is normally applied in amounts of from 0.01 to about 20 pounds per acre, preferably from 2 to 6 pounds per acre (0,4 acre ≈ 1 ha; 0,45 pound ≈ 1 Kg).

N-Phosphonomethylglycine, and certain soluble salts thereof, can be made in a number of different ways. One such method, as described in U.S. Patent 3,160,632 (Toy et al., December 8, 1964) is to react N-phosphinomethylglycine (glycinemethylenephosphinic acid) with mercuric chloride in a water solvent at reflux temperature, and subsequently separating the reaction products. Another method is the reaction of ethyl glycinate with formaldehyde and diethylphosphite. The latter method is described in U.S. Patent No. 3,799,758 (Franz, March 26, 1974). In addition, there is a whole series of patents, relating to N-phosphonomethylglycine, its salts, and derivatives thereof, described as being useful herbicides and plant growth regulators. Such additional patents relating to N-phosphonomethylglycine, methods of application, methods of preparation, salts, and derivatives, include U.S. Patent 3,868,407, U.S. Patent 4,197,254, U.S. Patent 4,199,354, and C4—A—620 223.

Because of the importance of N-phosphonomethylglycine and certain salts as a herbicide, other methods of making the compounds are constantly being sought in order to provide improved or alternate methods of manufacture.

It has now been discovered that N-phosphonomethylglycine can be produced by:

(a) reacting hydantoin or a 3-substituted hydantoin, compounds of the formula

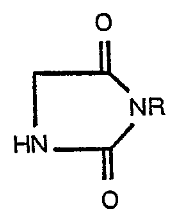

wherein R is selected from the group consisting of hydrogen, alkyl having from 1 to 10 carbon atoms, aryl having from 6 to 12 carbon atoms, alkylcarbonyl wherein the alkyl group has from 1 to 10 carbon atoms, and arylcarbonyl wherein the aryl group has from 6 to 12 carbon atoms, with paraformaldehyde in the presence of a low molecular weight carboxylic acid at a temperature and for a sufficient period of time to produce a mixture of intermediate products, including the 1-(hydroxymethyl) derivative;

(b) converting said 1-(hydroxymethyl) derivative to 1-phosphonomethylhydantoin by thereafter adding to the reaction mixture either

(i) a substituted phosphorus compound selected from the group consisting of phosphorus trichloride, and phosphorus tribromide; or

(ii) phosphorus acid and a carboxylic acid anhydride selected from the group consisting acetic anhydride, propionic anhydride, butyric anhydride, or mixtures thereof;

and continuing said reaction at a temperature and for a sufficient period of time to cause completion of the reaction to form the 1-(phosphonomethyl) derivative; and

(c) hydrolyzing said 1-(phosphonomethyl) derivative thus formed with an aqueous solution

of a base selected from the group consisting of alkali metal or alkaline earth hydroxide, to produce a salt of N-phosphonomethylglycine; and

(d) neutralizing said salt with a strong acid to produce the end product, N-phosphonomethylglycine.

The starting compound for use in the process of the invention is hydantoin, or a 3-substituted hydantoin, whose formula is indicated above. The preferred starting compound is the unsubstituted hydantoin, but 3-substituted hydantoins can be used, such as 3-methylhydantoin and 3-ethylhydantoin. Also suitable for use would be other alkyl-substituted hydantoins at the 3-position having from 1 to 20 carbon atoms, the aryl-substituted hydantoins at the 3-position having from 6 to 12 carbon atoms, the alkylcarbonyl-substituted hydantoins at the 3-position having from 1 to 10 carbon atoms in the alkyl group, and the arylcarbonylsubstituted hydantoins at the 3-position having from 6 to 12 carbon atoms in the aryl group. The substituted compounds are less preferred, however, because of their greater cost.

Paraformaldehyde is used in the process of the invention in order to eliminate water from the initial steps of the reaction. Aqueous formalin is obviously unsuitable because water is undesirable.

Acetic acid is the preferred low molecular weight carboxylic acid for use in step (a) of the process of the invention. Other suitable low molecular weight carboxylic acids which can be used include propanoic acid and butanoic acid, for example.

The most preferred substituted phosphorus compound for use in the above process is phosphorus trichloride. Other phosphorus compounds which can be used include phosphorus tribromide.

The preferred base for use in step (c) of the process is sodium hydroxide, however, other bases such as potassium hydroxide or barium hydroxide could also be used.

The preferred anhydride for use in step (b) (ii) is acetic anhydride. Other suitable anhydrides which can be used, however, include propionic anhydride, butyric anhydride, and mixed anhydrides of acetic, propionic, and butyric acids.

The preferred acid for use in step (d) of the process of the invention is hydrochloric acid, however, other acids such as hydrobromic acid, hydroiodic acid, sulfuric acid, and phosphoric acid, can be used. These acids may be described as relatively strong protic acids and any other acids falling within that purview would be suitable for use also.

Using the preferred compounds the overall reaction for the process of the invention can be presented as follows:

a) 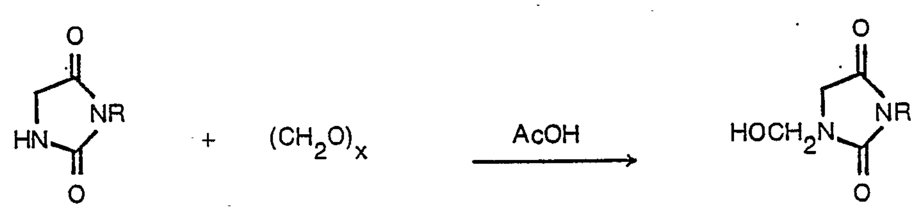


hydantoin paraformaldehyde hydroxymethyl hydantoin

b) (i) $PCl_3$ + 6 AcOH → $3Ac_2O$ + $HP(OH)_2$ + 3HCl

phosphorus trichloride, acetic acid, acetic anhydride, phosphorous acid, hydrogen-chloride

and/or

b) (ii) 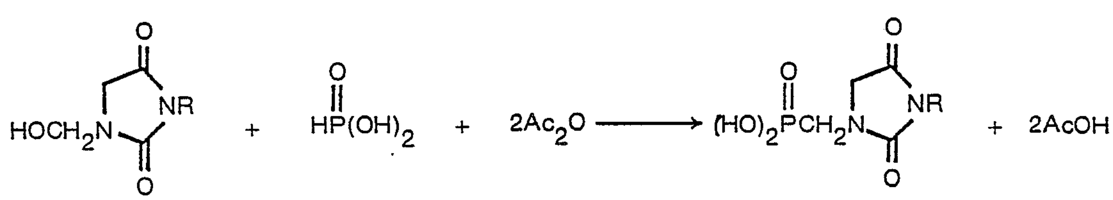

hydroxymethyl hydantoin, phosphorous acid, acetic anhydride, 1-phosphonomethyl hydantoin, acetic acid

c) 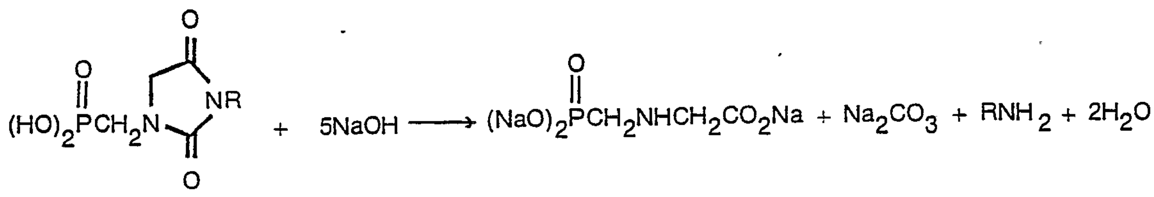

1-phosphonomethyl hydantoin

N-phosphonomethylglycine trisodium salt

d) $$(NaO)_2\overset{O}{\overset{\|}{P}}CH_2NHCH_2CO_2Na + 3HCl \longrightarrow \text{N-phosphonomethylglycine} + 3NaCl$$

In the process of the invention, the starting hydantoin compound, paraformaldehyde, and phosphorus trichloride are all used at approximately stoichiometric amounts in a ratio of 1:1:1. The low molecular weight carboxylic acid is generally present in excess. The same applies for the sodium hydroxide used in step (c), and in step (d), sufficient acid is used to bring the pH of the reaction mixture down to approximately 1.4, which is the pH at which the end product, N-phosphonomethylglycine, crystallizes most efficiently out of solution.

The first step of the reaction is conducted at reflux temperature, and when acetic acid is used as the carboxylic acid, this temperature ranges from approximately 115 to 120°C. The hydantoin and formaldehyde are refluxed for approximately 45 minutes. However, the exact amount of time is not critical.

The phosphorus trichloride in step (b) is initially added to the reaction mixture at room temperature and is then heated to reflux. Hydrogen chloride is evolved as the reactants are heated, thus, the temperature is raised slowly over a period of approximately 50 minutes until reflux is reached, again being approximately between 115 and 120°C. After the reflux temperature is reached, the reactants are refluxed for an additional 20 minutes to insure completion of the reaction.

While not shown in the formulas, water can optionally be added at the end of step (b) while the reactants are at reflux temperature. After water is added, the reaction mixture is refluxed for approximately 2 hours. The bulk of the carboxylic acid-water solvent mixture is then removed by evaporation at reduced pressure.

The base of step (c) is added at room temperature and the hydrolysis step is conducted at reflux (approximately 102°C) for a period of approximately 24 hours, when sodium hydroxide is used as the base of choice.

It has been found that the yield of phosphonomethylhydantoin can be improved if acetic anhydride or acetyl chloride is added at the very beginning of step (a) along with the paraformaldehyde and hydantoin. The reason for the improvement in yield is not exactly known. However, this improvement is only obtained when hydantoin is used rather than a 3-substituted derivative. Thus, when hydantoin is used, the preferred process includes the addition of acetic anhydride or acetyl chloride along with the other components in step (a) at the beginning of the reaction.

This invention will be better understood by reference to the specific example which follows, which serves to illustrate the instant invention.

## Example I

Preparation of 1-(Phosphono)hydantoin

A 500 milliliter (ml), round-bottom flask was equipped with a thermometer, condenser, magnetic stirrer and heating mantle. Into this flask was charged, under nitrogen, 10 g (0.10 mole) of hydantoin and 3.2 g (0.10) mole of paraformaldehyde (ca. 95% pure), along with 60 ml of anhydrous acetic acid. The mixture was then heated to reflux and maintained at reflux for 45 minutes. Thereafter, the reaction mixture was cooled to room temperature and at that time 13.8 g (0.10 mole) of phosphorus trichloride was charged all at once. An exotherm occurred. The reaction mixture was slowly heated to reflux while gas evolved. During the heating period a white precipitate (1,1′-methane-bis(hydantoin)) formed and then redissolved. The reaction mixture was kept at reflux for approximately 2 hours, and thereafter 150 ml of water was added and the mixture refluxed for an additional 1.5 hours. The mixture was then vacuum stripped at 70°C to give crude 1-(phosphonomethyl)hydantoin as 24.4 g of a viscous pink oil. The crude product was assayed quantitatively by high performance liquid chromatography (hplc) and found to be 43.7% pure. The yield, then, was 10.7 g, 56% of theory.

## Example II

Preparation of N-Phosphonomethylglycine from 1-(Phosphonomethyl)hydantoin

Crude 1-(phosphonomethyl)hydantoin was prepared as described above, induced to crystallize at low temperature, and then partially purified by digestion in refluxing isopropyl alcohol-ether (1:5). The purifed material was assayed by hplc

on an anion exchange column and found to be 75.3 weight % pure. A solution of 1.94 g (7.53 mmole) of this material and 50 ml 2 *N* aqueous NaOH was heated at reflux (102°C) for 24 hours. The cooled reaction mixture was assayed by hplc analysis on an anion exchange column and found to contain 7.65 ± 0.40 mmole of N-phosphonomethylglycine trisodium salt. The yield of the salt, therefore, was approximately 100%.

The solution of the salt was acidified with 12 *N* HCl to pH 1.5 and filtered to remove precipitated white solids (probably silicic acid). THe filtrate was chilled, seeded, and allowed to stand at ca 5°C for a few days. A precipitate of crystalline phosphonomethylglycine formed and was isolated by filtration, washing, and drying. The yield was 0.48 g (1.84 mmol, 38% based on 1-phosphonomethylglycine). The nmr and ir spectra of the product were identical to those of authentic material.

Example III

Preparation of 1-(Phosphonomethyl)hydantoin

The preparation of 1-(phosphonomethyl)-hydantoin was carried out by a slight modification of the method of Example I. The only change made was that 11.2 g (0.110 mole) of acetic anhydride was added at the outset. The yield of 1-(phosphonomethyl)hydantoin was 76% of the theoretical amount.

Example IV

Preparation of 1-(Phosphonomethyl)hydantoin

The preparation was carried out by a slight modification of the method of Example I. The change made was that 8.6 g (0.110 mole) of acetyl chloride was added at the outset. This made it necessary to heat the initial mixture with caution, because a strongly exothermic reaction, accompanied by gas evolution, occurred when heating was begun. In all other respects the method used was the same as that of Example I. The yield of 1-(phoshonomethyl)hydantoin was 75% of the theoretical amount.

Example V

Preparation of 1-(Phosphonomethyl)hydantoin

A 500 ml round-bottom flask was equipped with a thermometer, condenser, magnetic stirrer, and heating mantle. Into the flask was charged, under nitrogen, 10.0 g (0.100 mole) of hydantoin, 3.2 g (0.10 mole) of paraformaldehyde (purity: ca 95%), and 60 ml of anhydrous acetic acid. The resulting mixture was heated at reflux for 0.75 hour and then cooled. To the resulting clear solution was added 8.5 g (0.100 mole) of 97% phosphorus acid and 30.6 g (0.300 mole) of acetic anhydride. The reaction mixture was heated to reflux over 1.2 hours and held at reflux for another 0.1 hour. (A white solid precipitated early in the heating period and then redissolved toward the end). The reaction mixture was cooled somewhat, and 150 ml of water was added to it. The resulting solution was heated at reflux for 2.0 hours, cooled, and vacuum stripped at 70°C to give 21.4 g of crude 1-(phosphonomethyl)hydantoin as an oil. Quantitative analysis by hplc showed the yield of 1-(phosphonomethyl)hydantoin to be 46%.

Example VI

N-Phosphonomethylglycine Trisodium Salt

Crude 3-methyl-1-(phosphonomethyl)hydantoin (6.54 g) was prepared by the method of Example I from 2.85 g (0.025 mole) of 3-methylhydantoin, 0.8 g (ca 0.025 mole) of paraformaldehyde, 11 ml of anhydrous acetic acid, and 3.5 g (0.024 mole) of phosphorus trichloride. A portion (5.56 g) of the crystalline crude product was dissolved in 10 ml of water, and the solution was basified to pH 10.7 with 20% aqueous sodium hydroxide. To the resulting solution was added 200 ml of 2 *N* sodium hydroxide. The solution obtained was heated at reflux for 24 hours, cooled, weighed and quantitatively assayed by hplc for N-phosphonomethylglycine, trisodium salt. The yield was found to be 72% of the theoretical amount.

In carrying out the process of the invention, it should be noted that after phosphorus trichloride is added to the reaction mixture, a white precipitate normally crystallizes out, but is later dissolved when the solution is heated to reflux temperature. The crystalline compound has been isolated and found to be 1,1′-methane-bis-hydantoin. It has been found that this substance is converted to 1-(phosphonomethyl)hydantoin (49% yield) when it is treated with phosphorus trichloride and acetic acid and the reaction mixture is worked up in the usual way.

In step (b) of the process of the invention, it makes little difference whether sub-section (i) or (ii) is follwed because the end product is the same in both instances. Thus, when the phosphorus trichloride is added and used in conjuction with acetic acid, the intermediate product produced is 1-(phosphonomethyl)hydantoin, while when section (a) (ii) is used, and acetic anhydride and phosphorus acid are combined, the product is still 1-(phosphonomethyl)hydantoin. It is believed that the phosphorus trichloride and acetic acid react in solution to give phosphorous acid and acetic anhydride, the components used in step (b) (ii) as shown above.

**Claims**

1. A process for the production of N-phosphonomethylglycine characterised in that it comprises:

(a) reacting hydantoin or a 3-substituted hydantoin corresponding to the following general formula:

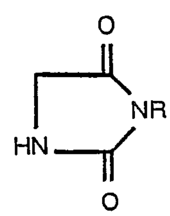

wherein R represents hydrogen, $C_1$—$C_{10}$ alkyl, $C_6$—$C_{12}$ aryl, $C_1$—$C_{10}$ alkyl-carbonyl and $C_6$—$C_{12}$ aryl-carbonyl with paraformaldehyde in the presence of a low molecular weight carboxylic acid to produce a mixture of intermediate products including the 1-(hydroxymethyl) derivative of the starting hydantoin;

(b) converting the 1-(hydroxymethyl) derivative to the 1-phosphonomethyl derivative by thereafter adding to the reaction mixture either:

(i) a substituted phosphorus compound selected from phosphorus trichloride and phosphorus tribromide; or

(ii) phosphorus acid and an anhydride selected from acetic anhydride, propionic anhydride, butyric anhydride or mixtures thereof and continuing the reaction to cause completion of the reaction to form the 1-phosphonomethyl derivative;

(c) hydrolyzing the resulting 1-phosphonomethylhydantoin product with a base selected from alkali metal or alkaline earth hydroxides to produce a salt of N-phosphonomethylglycine; and

(d) neutralizing the salt with a strong acid.

2. A process as claimed in claim 1 wherein in (b), the substituted phosphorus compound is phosphorus trichloride.

3. A process as claimed in claim 1 or claim 2 wherein, in (a), the low molecular weight carboxylic acid is selected from acetic, propanoic and butanoic acids.

4. A process as claimed in any of claims 1 to 3 wherein, in (a), the hydantoin is 3-methyl hydantoin.

5. A process as claimed in any of claims 1 to 4 wherein the hydantoin, paraformaldehyde and substituted phosphorus compound are used in approximately stoichiometric amounts and the carboxylic acid is used in excess.

6. A process as claimed in any of claims 1 to 4 wherein the paraformaldehyde is used in excess relative to the hydantoin.

7. A process as claimed in any of claims 1 to 6 wherein, in (d), the acid is selected from hydrochloric acid, sulfuric acid, hydrobromic acid and hydroiodic acid.

8. A process as claimed in any of claims 1 to 7 wherein, in (c), the base is sodium hydroxide.

9. A process as claimed in any of claims 1 to 8 wherein, in (a), acetyl chloride or acetic anhydride is also added, when hydantoin is used.

10. A process as claimed in any of claims 1 to 9 wherein in (b), phosphorus acid and an anhydride selected from acetic anhydride, propionic anhydride, butyric anhydride or mixtures thereof are added to the reaction mixture.

**Patentansprüche**

1. Verfahren zur Herstellung von N-Phosphonomethylglycin charakterisiert durch:

(a) Reaktion von Hydantoin oder in 3-Stellung substituiertem Hydantoin entsprechend der folgenden allgemeinen Formel

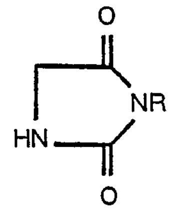

wobei R Wasserstoff, eine $C_1$—$C_{10}$ Alkyl-, $C_6$—$C_{12}$ Aryl-, $C_1$—$C_{10}$ Alkylcarbonyl- oder $C_6$—$C_{12}$ Arylcarbonyl-Gruppen darstellt, mit Paraformaldehyd in Gegenwart einer Carbonsäure mit niedrigem Molekulargewicht, so daß ein Gemisch von Zwischenprodukten entsteht, das das 1-Hydroxymethylderivat des als Ausgangsmaterial verwendeten Hydantoins enthält;

(b) Umwandlung dieses 1-Hydroxymethylderivates zum 1-Phosphonomethylderivat dadurch, daß anschließend dem Reaktionsgemisch entweder

(i) eine substituierte Phosphorverbindung, und zwar Phosphortrichloid oder Phosphortribromid oder

(ii) phosphorige Säure und ein Säureanhydrid, and zwar Essigsäureanhydrid oder Propionsäureanhydrid oder Buttersäureanhydrid oder deren Gemische

zugesetzt und die Reaktion weitergeführt wird, so daß nach Vervollständigung der Reaktion das 1-Phosphonomethylderivat gebildet wird;

(c) Hydrolyse des erhaltenen 1-Phosphonomethylhydantoins mit einem Alkalimetall- oder Erdalkalimetallhydroxid als Base, so daß N-Phosphonomethylglycin als Salz gebildet wird, und

(d) Neutralisation des Salzes mit einer starken Säure.

2. Verfahren nach Anspruch 1, dadurch gekennzeichnet, daß in (b) die substituierte Phosphorverbindung Phosphortrichlorid ist.

3. Verfahren nach einem der Ansprüche 1 oder 2, dadurch gekennzeichnet daß in (a) die Carbonsäure mit niedrigem Molekulargewicht Essigsäure oder Propionsäure oder Buttersäure ist.

4. Verfahren nach einem der Ansprüche 1 bis 3, dadurch gekennzeichnet, daß in (a) das verwendete Hydantoinderivat 3-Methylhydantoin ist.

5. Verfahren nach einem der Ansprüche 1 bis 4, dadurch gekennzeichnet, daß das Hydantoin, Paraformaldehyd und die substituierte Phosphorverbindung in etwa stöchiometrischem Verhältnis und die verwendete Carbonsäure im Überschuß eingesetzt werden.

6. Verfahren nach einem der Ansprüche 1 bis 4, dadurch gekennzeichnet, daß Paraformaldehyd im Überschuß bezogen auf das Hydantoin eingesetzt wird.

7. Verfahren nach einem der Ansprüche 1 bis 6, dadurch gekennzeichnet, daß in (d) die Säure Salzsäure, Schwefelsäure, Bromwasserstoffsäure oder Jodwasserstoffsäure ist.

8. Verfahren nach einem der Ansprüche 1 bis 7, dadurch gekennzeichnet, daß in (c) die Base Natriumhydroxyd ist.

9. Verfahren nach einem der Ansprüche 1 bis 8, dadurch gekennzeichnet, daß in (a) Acetylchlorid oder Essigsäureanhydrid ebenfalls zugesetzt wird, wenn Hydantoin eingesetzt wird.

10. Verfahren nach einem der Ansprüche 1 bis 9, dadurch gekennzeichnet, daß in (b) phosphorige Säure und ein Säureanhydrid, und zwar Essigsäureanhydrid oder Propionsäureanhydrid oder Buttersäureanhydrid oder deren Gemische zum Reaktionsgemisch zugesetzt werden.

**Revendications**

1. Un procédé de production de N-phosphonométhylglycine caractérisé en ce qu'il consiste:

a. à faire réagir une hydantoïne 3-substituée, correspondant à la formule générale:

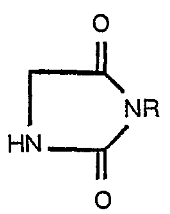

dans laquelle R représente un atome d'hydrogène, un radical alkyle comprenant 1 à 10 atomes de carbone, un radical aryle comprenant 6 à 12 atomes de carbone, un radical alkylcarbonyle comprenant 1 à 10 atomes de carbone ou un radical arylcarbonyle comprenant 6 à 12 atomes de carbone, avec du paraformadéhyde en présence d'un acide carboxylique de bas poids moléculaire pour produire un mélange des produits intermédiaires incluant le dérivé 1-(hydroxyméthyle) de l'hydantoïne de départ;

b. à convertir le dérivé hydroxyméthyle en un dérivé phosphonométhyle par addition ultérieure au mélange réactionnnel:

(i) soit d'un composé du phosphore substitué choisi dans le groupe comprenant le trichlorure de phosphore et le tribromure de phosphore,

(ii) soit de l'acide phosphorique et un anhydride choisi parmi l'anhydride acétique, l'anhydride propionique, l'anhydride butyrique ou leurs mélanges;

et continuer cette réaction pour conduire à l'achèvement de la réaction de formation du dérivé 1-phosphonométhyle;

c. à hydrolyser 1-(phosphonométhyl)-hydantoïne résultant avec une base choisie parmi les hydroxydes de métaux alcalins ou alcalino-terreux pour produire un sel de N-phosphonométhylglycine; et

d. à neutraliser le sel à l'aide d'un acide fort.

2. Un procédé ainsi que revendiqué dans la revendication 1, dans lequel dans (b), un composé de phosphore substitué est le trichlorure de phosphore.

3. Un procédé ainsi que revendiqué dans la revendication 1 ou dans la revendication 2, dans lequel dans (a), l'acide carboxylique de bas poids moléculaire est choisi parmi les acides acétique, propanoïque et butanoïque.

4. Un procédé ainsi que revendiqué dans l'une quelconque des revendications 1 à 3, dans lequel dans (a), l'hydantoïne est la 3-méthylhydantoïne.

5. Un procédé ainsi que revendiqué dans l'une quelconque des revendications 1 à 4, dans lequel dans (a), l'hydantoïne, le paraformaldéhyde et le composé de phosphore substitué sont utilisés en quantités approximativement stoéchiométriques et dans lequel, l'acide carboxylique étant utilisé en excès.

6. Un procédé ainsi que revendiqué dans l'une quelconque des revendications 1 à 4, dans lequel le paraformaldéhyde est utilisé en excès par rapport à l'hydantoïne.

7. Un procédé ainsi que revendiqué dans l'une quelconque des revendications 1 à 6, dans lequel dans (d) l'acide est choisi parmi l'acide chlorhydrique, l'acide sulfurique, l'acide bromhydrique et l'acide iodhydrique.

8. Un procédé ainsi que revendiqué dans l'une quelconque des revendications 1 à 7, dans lequel dans (c) la base est l'hydroxyde de sodium.

9. Un procédé ainsi que revendiqué dans l'une quelconque des revendications 1 à 8, dans lequel dans (a) le chlorure d'acétyle ou l'anhydride acétique sont également utilisés lorsque l'on utilise de l'hydantoïne.

10. Un procédé ainsi que revendiqué dans l'une quelconque des revendications 1 à 9, dans lequel dans (b) ou ajoute, au mélange réactionnel, de l'acide phosphorique et un anhydride choisi parmi l'anhydride acétique, propionique, butyrique ou leurs mélanges.